# EUROPÄISCHE PATENTSCHRIFT

(11) **EP 2 763 657 B1**
(45) Veröffentlichungstag und Bekanntmachung des Hinweises auf die Patenterteilung: **25.03.2020**
(21) Anmeldenummer: 12791077.6
(22) Anmeldetag: 01.10.2012
(51) Int. Cl.: A61K 9/127, A61K 45/06, A61K 31/05, A61K 31/121, A61K 31/353

(54) **MOLEKÜLMISCHUNG, UMFASSEND EINE AMPHIPATHISCHE MOLEKÜLSORTE A, WELCHE IM HYDROPHILEN BEREICH EINE POSITIVE GESAMTLADUNG AUFWEIST UND EINE AMPHIPATHISCHE MOLEKÜLSORTE B SOWIE EIN POLYPHENOL C, VERFAHREN ZUR HERSTELLUNG DER MOLEKÜLMISCHUNG UND DEREN VERWENDUNG**
MOLECULE MIXTURE COMPRISING AN AMPHIPATHIC MOLECULE TYPE A, WHICH HAS A POSITIVE TOTAL CHARGE IN THE HYDROPHILIC RANGE, AND AN AMPHIPATHIC MOLECULE TYPE B AND A POLYPHENOL C, METHOD FOR PRODUCING SAID MOLECULE MIXTURE AND USE THEREOF
MÉLANGE DE MOLÉCULES COMPRENANT UN TYPE DE MOLÉCULE AMPHIPATHIQUE A PRÉSENTANT UNE CHARGE TOTALE POSITIVE DANS LA ZONE HYDROPHILE ET UN TYPE DE MOLÉCULE AMPHIPATHIQUE B, AINSI QU'UN POLYPHÉNOL C, PROCÉDÉ DE PRÉPARATION DUDIT MÉLANGE DE MOLÉCULES ET SON UTILISATION

(30) Priorität: 06.10.2011 DE 102011114951
(43) Veröffentlichungstag der Anmeldung: 13.08.2014
(73) Patentinhaber: Forschungszentrum Jülich GmbH, 52425 Jülich (DE)
(72) Erfinder: CSISZAR, Agnes, 52428 Jülich (DE); KLEUSCH, Christian, 53123 Bonn (DE); HOFFMANN, Bernd, 52428 Jülich (DE); MERKEL, Rudolf, 52428 Jülich (DE)
(86) Internationale Anmeldenummer: PCT/DE2012/000956
(87) Internationale Veröffentlichungsnummer: WO 2013/050017

(56) Entgegenhaltungen:
- EP-A1- 2 123 259
- EP-A2- 2 451 441
- US-A1- 2008 138 400
- US-A1- 2009 081 282
- US-A1- 2010 119 593

## Beschreibung

Molekülmischung, umfassend eine amphipathische Molekülsorte A, welche im hydrophilen Bereich eine positive Gesamtladung aufweist und eine amphipathische Molekülsorte B sowie ein Polyphenol C, Verfahren zur Herstellung der Molekülmischung und deren Verwendung Die Erfindung bezieht sich auf eine Molekülmischung, umfassend eine amphipathische Molekülsorte A, welche im hydrophilen Bereich eine positive Gesamtladung aufweist und eine amphipathische Molekülsorte B sowie ein Polyphenol C, deren molekulares Verhältnis zueinander, das Verfahren zur Herstellung der Molekülmischung und deren Verwendung.

### Stand der Technik

Polyphenole sind sekundäre Pflanzenstoffe, die in Pflanzensamen, in Blättern und in Früchten vorkommen. Einige dieser Stoffe wirken auf Krebszellen hemmend (Mentha, R.G., Murillo G., Naithani R. and Peng X., Pharm Res (2010) 24:950-961) oder schützen vor unerwünschten Oxidationsprozessen (Queen B.L., Tollefsbold T.O., Curr. Aging Sci. (2010). 3:34-42). Daher zählen sie zu den wichtigen Bestandteilen menschlicher Ernährung. Auf der anderen Seite sind sie auch wichtige Bestandteile in der Chemotherapie, da sie bei extrem hohen Konzentrationen eine toxische Wirkung aufweisen. Aufgrund der hohen therapeutischen Relevanz der Polyphenole ist deren kontrollierte und effektive Verabreichung an Lebewesen von großem Interesse.

Die Verabreichung solcher hydrophober Wirkstoffe in wässrigen Lösungen hat mehrere Nachteile. Zum einen wird eine sehr hohe Konzentration benötigt, um eine therapeutische Wirkung zu erzielen. Der größte Teil des Wirkstoffes liegt für die Zellen nicht erreichbar vor und daher bleiben ca. 99 % der Polyphenole ohne zelluläre Aufnahme. Zum Anderen verlieren sie durch unerwünschte Oxidation in der wässrigen Umgebung ihre Wirkung.

Das Dokument US 2008/138400 A1 offenbart liposomales Curcumin und dessen Herstellung. Das Dokument EP 2123259 A1 offenbart ein Liposom enthaltend unter amderem ein kationisches Lipid und eine antioxidative Komponente, wie beispielsweise Curcumin.

Das Dokument US 2009/081282 A1 offenbart ein Liposom enthaltend ein kationisches Lipid und ein amphiphiles, neutrales Lipid im Verhältnis 1:1. Als Medikament kann das Liposom Curcumin enthalten.

Liposomen und Polymerkapseln erhöhen die Bioverfügbarkeit der Polyphenole, da diese eher den physiko-chemischen Charakter und die Hydrophylie im Vergleich zur wässrigen Verabreichung aufweisen (Nair H.B. et al., Biochem. Pharmacology (2010). 80:1833-1843; Kristl J. et al., Eu. J. of Pharmaceutics and Biopharmaceutics (2009). 73:253-259; Narayanan N.K., Nargi D., Randolph C., and Narayanan B.A., (2009). Int. J. Cancer. 125: 1-8). Obwohl diese Kapseln somit Vorteile haben, ist nachteilig eine kontrollierte Abgabe und schnelle Aufnahme des Polyphenols durch die Zielzellen nicht gewährleistet. Auch können diese Liposomen das Polyphenol nur zum Teil gegen unerwünschte Oxidationsprozesse schützen.

### Aufgabe und Lösung der Erfindung

Aufgabe der Erfindung ist es daher, eine Molekülmischung bereit zu stellen, in der das Polyphenol stabilisiert und vor Oxidationsprozessen geschützt vorliegt. Das Polyphenol oder eine Mischung an Polyphenolen soll derartig vorliegen, dass es schnell und mit hoher Effizienz auch in überdurchschnittlich hohen Menge kontrolliert, das heißt in der gewünschten Menge in lebende Zellen eingebracht werden kann und dadurch entweder eine gezielt präventive oder aber eine gezielt toxische Wirkung auf die Zelle zu erzielen. Ferner ist es die Aufgabe der Erfindung ein Verfahren zur Herstellung der Mischung und deren Verwendung anzugeben.

### Beschreibung der Erfindung

Die Aufgabe der Erfindung wird durch die erfindungsgemäße Mischung nach dem Hauptanspruch, das Verfahren zur Herstellung der Mischung und die Verwendung der Mischung gelöst. Vorteilhafte Ausgestaltungen ergeben sich aus den jeweils darauf rückbezogenen Ansprüchen und insbesondere aus dem Patentanspruch 6, der die Molekülmischung als Liposom beansprucht.

Die erfindungsgemäße Molekülmischung weist mindestens zwei Molekülsorten A und B sowie das Polyphenol C auf.

Die beiden Molekülsorten A und B liegen in einem molekularen Verhältnis von A:B von etwa 1:1 vor. Geringfügige Abweichungen von dem bevorzugten Verhältnis von 1:1 mol/mol sind zuzulassen, das heißt A:B von 1(±0,2):1(±0,2) mol/mol.

Das Molverhältnis der Molekülsorten A, B und des Polyphenols C soll 1(±0,2):1(±0,2):0,5-5 mol/mol betragen. Für diesen Fall ist in vitro der Nachweis des Einbaus des Polyphenols C in die Zielzelle über ein Liposom bereits erfolgt.

Geringfügige Abweichungen von dem Verhältnis hiervon sind wie erwähnt erlaubt, soweit die sich abstoßenden Ladungen der Molekülsorte A durch die neutralen Hilfsmoleküle B etwa ausgeglichen werden. Das Verhältnis aus A:B:C wird für das Polyphenol C ab etwa 1:1:5 mol/mol in vitro hin zu toxischen Konzentrationen verschoben.

Es versteht sich, dass die entsprechenden Zwischenwerte für die jeweilige Molekülsorte A und B zuzulassen sind, das heißt z. B. jeweils 0,8, 0,9, 1,0, 1,1 und 1,2 für die Molekülsorte A und die Molekülsorte B und wiederum deren Zwischenwerte. Das Gleiche gilt für das Polyphenol C, sofern nur gewährleistet ist, dass eine schnelle Inkorporation des Polyphenols C in die Zielzelle aus einer Mischung heraus und insbesondere aus dem Liposom in die Zelle gewährleistet ist.

Im Rahmen der Erfindung wurde weiterhin erkannt, dass Polyphenole mittels positiv geladener Liposome, die aus den erfindungsgemäßen Mischungen heraus hergestellt werden, viel besser in Zielzellen und in deren Membran eingeschlossen und inkorporiert werden können, als wenn man von neutralen Liposomen ausgeht, wie dies im angezeigten Stand der Technik nach Kristl et al. getan wird. Dort ist bedingt durch das Herstellungsverfahren nur eine geringe Beladung des Liposoms mit Resveratrol weitgehend auf der Oberfläche des fertigen Liposoms erfolgt, da das Polyphenol dort erst nach der Herstellung des Liposoms in wässriger Phase hinzugegeben wird. In dem erfindungsgemäßen Fall ist das Polyphenol hingegen bereits bei der Herstellung des Liposoms aus der getrockneten Mischung zugegen. Das Polyphenol stellt daher einen essentiellen Bestandteil in der Liposommembran dar und wird in hohem Maße in diese eingebaut. Dadurch erfolgt vorteilhaft eine kontrollierte, gezielte, vollständige Beladung des Liposoms mit dem Polyphenol C. Darüber hinaus wurde im Rahmen der Erfindung erkannt, dass das Polyphenol in der Liposommembran dann selbst Fusion auslösend mit der Membran der Zelle ist. Dadurch wird vorteilhaft bewirkt, dass das Polyphenol mit einer hohen Effizienz schnell in die Zielzellen überführt wird.

Im Übrigen wurde festgestellt, dass das Polyphenol in der Membran des sich bildenden Liposoms sehr von vorläufigen Oxidationsprozessen geschützt vorliegt.

Für das Herstellungsverfahren der Mischung wurde erkannt, dass Polyphenole generell gut löslich in organischen Lösungsmitteln, wie Ethanol oder Chloroform sind und diese deshalb eine vorläufige Oxidation der Polyphenole unterbinden können. Eine Mischung des gelösten Polyphenols mit den neutralen und positiv geladenen Lipiden der Molekülsorten A und B in mindestens einem organischen Lösungsmittel wird daher zur Bereitstellung der erfindungsgemäßen Mischung durchgeführt. Dies dient der Homogenisierung der Molekülmischung.

Es versteht sich, dass die Mischung nach Entfernung des organischen Lösungsmittels vorteilhaft in pulverförmiger, homogenisierter Form vorliegt. Dies allein löst bereits die Aufgabe der Erfindung.

Wie für die Mischung soll für das Herstellungsverfahren entsprechend als molekulares Verhältnis A:B von 1(±0,2):1(±0,2) mol/mol gewählt werden. Das Molverhältnis der positiven und neutralen Lipide der Molekülsorten A, B und des Polyphenols C soll 1(±0,2):1(±0,2):0,5-5 mol/mol betragen bzw. eingestellt werden. Geringfügige Abweichungen hiervon sind wie erwähnt erlaubt.

Die erfindungsgemäße Mischung, die vorzugsweise als Liposom definiert ist und das zur Anwendung gelangende Medikament auf Basis der erfindungsgemäßen Mischung, insbesondere des Liposoms, umfasst somit die Molekülsorte A und eine Molekülsorte B sowie ein Polyphenol C im angegebenen Verhältnis.

In einer weiteren besonders vorteilhaften Ausgestaltung der Erfindung wird durch die Überführung von der organischen Phase über die pulverförmige Phase in die wässrige, liposomale Umgebung in besonders vorteilhafter Weise die Bioverfügbarkeit der Polyphenole für therapeutische Zwecke bei gleichzeitigem Schutz vor Oxidation erhöht.

Die erfindungsgemäße Molekülmischung aus den Molekülsorten A und B mit dem Polyphenol C wird somit von dem organischen Lösungsmittel bzw. den organischen Lösungsmitteln getrennt und schonend in einen wässrigen Puffer um pH 7 überführt. Das heißt, dass zur Herstellung des Liposoms im Wesentlichen die organische Komponente gegen eine auf wässrigem, physiologischem Puffer beruhenden System ausgetauscht wird.

Erst in der wässrigen Umgebung bilden sich die besonders bevorzugten multilamellaren, in sich geschlossenen sphärischen Strukturen, die sogenannten Liposome aus. Durch zusätzliche Schritte wie Ultraschallbehandlung, Extrusion oder mehrfaches Abkühlen und Aufheizen der Lösungen auf bis zu 4°C bzw. auf bis zu 70 °C können die Liposomen weiter homogenisiert werden. Diese Strukturen weisen auf Grund der Molekülsorte A eine positive Ladung (Zeta Potential Uz=30-100 mV) auf. Die meisten apolaren Polyphenole befinden sich im Inneren der Lipiddoppelschichten, der Liposommembran und sind dadurch von reaktiven Sauerstoffradikalen in vorteilhafter Weise abgeschirmt und geschützt. Derartige Liposome sind aus allen erfindungsgemäßen Mischungen heraus zu bilden.

Diese mit Polyphenol beladenen, erfindungsgemäßen Liposome fusionieren aufgrund der Wechselwirkung der positiv geladenen Lipide und der delokalisierten Elektronen der pflanzlichen Polyphenole durch einfachen Oberflächenkontakt über wenige Minuten (5-20 min) hocheffizient mit der Plasmamembran tierischer Zellen.

Wenn die Mischung als Liposom vorliegt, so liegt das Polyphenol C zum größten Teil in der Liposommembran eingebaut vor. Nur zum kleineren Teil ist es im Innern des Liposoms selbst in wässrigem Puffer gelöst. Dies hat zur Folge, dass das Polyphenol selbst eine Fusion mit der Zielzellenmembran auslöst.

Die Phenole selber werden während der therapeutischen Anwendung anstelle der zellulären Moleküle oxidiert. Dadurch verhindert die Anwesenheit der Polyphenole vorteilhaft die Überoxidation der Zelle. Die erfindungsgemäße Mischung und das Medikament hieraus ist daher ein wirksames Therapeutikum bei der Behandlung aller oxidativen Krankheitsprozesse oder bei bestimmten Krebserkrankungen.

Wenn in vitro größere Mengen von diesen Radikalfängern in der Mischung bzw. im Liposom vorhanden sein sollen, können Werte mit einem Molverhältnis von etwa 1:1:5 mol/mol der Molekülsorten A, B und des Polyphenols C angewendet bzw. verabreicht werden. Diese Mischung wirkt dann eher toxisch. Daher wird diese hohe oder noch höhere Menge bei Krebstherapien vorgeschlagen, um Krebszellen in hohen Maßen zu überoxidieren und dadurch abzutöten. In vivo sind andere Konzentrationen möglich und in Betracht zu ziehen. Die Mischungen und positiv geladenen Liposomen müssen somit eine Molekülsorte A und eine Molekülsorte B beinhalten. Die Kriterien für die Molekülsorte A sind, dass (a) das Molekül einen hydrophilen Bereich mit mindestens einer oder mehreren positiven Ladungen aufweist, so dass die Gesamtladung des hydrophilen Teils des Moleküls positiv ist. Die Aufgabe dieses Moleküls ist es, die Fusionsmischung durch elektrostatische Kräfte in die Nähe der negativ geladenen Zellmembran zu bringen. (b) Molekülsorte A weist zudem einen hydrophoben Bereich auf, vorzugsweise einen C10-C30-Anteil mit oder ohne Doppelbindungen. Doppelbindungen haben die vorteilhafte Wirkung, dass die Membran des entstehenden Liposoms elastisch wird, so dass die Fusion des Liposoms mit der Zellmembran erleichtert wird. Geeignet sind Moleküle wie z.B. 1,2-Dioleoyl-3-Trimethylammonium-Propan (DOTAP), N-(2,3-Dioleyloxypropyl)-N, N, N-Trimethylammonium Chlorid (DOTMA), Dimethyl-Dioctadecyl Ammonium Bromid (DDAB) oder (1-[2-(Oleoyloxy)Ethyl]-2-Oleyl-3-(2-Hydroxyethyl)Imidazolinium Chlorid (DOTIM). Als ein erstes Beispiel wird DOTAP (1,2-Dioleoyl-3-Trimethylammonium-Propan (Chlorid-Salz)) genannt.

Die Kriterien für Molekülsorte B sind, dass (a) das Molekül einen hydrophilen Bereich sowie (b) einen hydrophoben Bereich (insbesondere C10-C30) mit oder ohne Doppelbindungen aufweisen muss. Zur Funktion der Doppelbindungen siehe Sorte A. (c) Beide Bereiche sollen einen neutralen Charakter aufweisen, um die große Ladungsdichte und die abstoßenden Kräfte zwischen positiv geladenen Molekülen von Molekülsorte A zu neutralisieren. Dieser Effekt führt zur Stabilisierung des Systems. Molekülsorte B ist somit ein Helfermolekül. Geeignet sind Moleküle wie z. B. Phosphatidylethanolamine und Phosphatidylcholine, wie z. B. 1,2-Dioleoyl-sn-Glycero-3-Phosphoethanolamin (DOPE), 1,2-Dipalmitoyl-sn-Glycero-3-Phosphoethanolamin (DPPE), 1,2-Dimiristoyl-sn-Glycero-3-Phosphoethanolamin (DMPE), 1,2-Dielaidoyl-sn-Glycero-3-Phosphoethanolamin (DEPE), 1,2-Diphytanol-sn-Glycero-3-Phosphoethanolamin, 1,2-Dilinoleoyl-sn-Glycero-3 Phosphoethanolamin oder 1,2-Dioleoyl-sn-Glycero-3-Phosphatidylcholin (DOPC).

Die verwendeten Polyphenole sind Moleküle, die mindestens zwei, direkt an einen aromatischen Ring gebundene Hydroxylgruppen enthalten und zu den sekundären Pflanzenstoffen gehören. Vorzugsweise werden für die erfindungsgemäße Mischung und Liposome Polyphenole, wie beispielweise Resveratrol, Curcumin, Hydroxyflavon und Genistein in dem angegebenen Verhältnis gewählt, wobei Hydroxyflavon kein Polyphenol in Sinne der Erfindung ist

Im Weiteren wird die Erfindung an Hand von Ausführungsbeispielen und der beigefügten zwei Figuren näher beschrieben, ohne dass es hierdurch zur Einschränkung der Erfindung kommen soll.

Es zeigen:
- Figur 1:: Strukturen der bevorzugten Polyphenole: I. Resveratrol. II. Curcumin. III. Hydroxyflavon. IV. Genistein, wobei Hydroxyflavon kein Polyphenol in Sinne der Erfindung ist.
- Figur 2:: Einbau von Resveratrol in positiv geladene Liposomen und dessen in vitro Nachweis in Zellen.

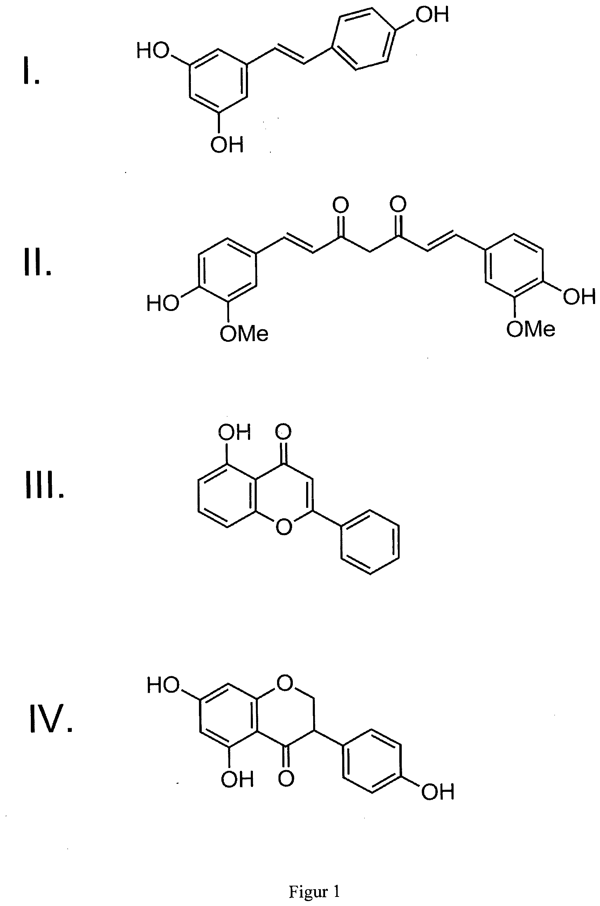

Die in der Figur 1 dargestellten Strukturformeln sind lediglich beispielhaft für einige besonders relevante und gut verfügbare Polyphenole gezeigt. Andere, hier nicht dargestellte Polyphenole sollen aber explizit mit von den geltenden Patentansprüchen umfasst sein, sofern sie der oben genannten Definition genügen. Es sind also selbstverständlich auch andere Polyphenole als die gezeigten zur Herstellung und Verwendung der erfindungsgemäßen Mischungen und Liposome denkbar.

### 1. Ausführungsbeispiel - in vitro Untersuchungen an Zellen:

1,2-Dioleoyl-3-Trimethylammonium-Propan, Chlorid-Salz (DOTAP) als positiv geladenes Lipid der Molekülsorte A sowie 1,2-Dioleoyl-sn-Glycero-3-Posphoethanolamin (DOPE) als neutrales Lipid der Molekülsorte B (Avanti Polar Lipids Inc., Alabaster, AL, USA) und N-(4,4-Difluoro-5,7-Dimethyl-4-bora-3a,4a-Diaza-s-indacene-3-Propionyl)-1,2-Dihexadecanoyl-sn-Glycero-3-Phosphoethanolamine (Triethylammonium-Salz) (entspricht BODIPY® FL-DHPE) (Invitrogen, Eugene, OR, USA) als fluoreszentes Markerlipid wurden in Chloroform/Ethanol (10/1 vol/vol) Lösungsmittel-Mischung in einem Molverhältnis von DOTAP/DOPE/BODIPY® FL-DHPE = 1/1/0,005 mol/mol in einer Gesamtlipidkonzentration von etwa 1 mg/ml aufgenommen und gemischt.

BODIPY® FL-DHPE dient hierbei lediglich zum Zwecke des Nachweises einer erfolgreichen Membranfusion zwischen Liposom und Plasmamembran. Derartige Farbstoffe sind explizit nicht Gegenstand der erfindungsgemäßen Mischung bzw. des erfindungsgemäßen Liposoms.

Resveratrol (Sigma-Aldrich, St. Louis, MO, USA) wurde zunächst in Ethanol als organisches Lösungsmittel in einer Konzentration von 1 mg/ml aufgelöst und homogenisiert.

Die Lipidkomponente aus Molekülsorte A und B und BODIPY® FL-DHPE wurde sodann mit Resveratrol in folgenden Molverhältnissen aus der organischen Lösungsmittelmischung heraus miteinander gemischt (Tabelle 1).

**Tabelle 1: Angaben zu Mengen und Volumina der Komponente A, B und des Polyphenols C sowie der Lösungsmittel (MW=Molekulargewicht; stock=Stammlösung).**

| Komponente | MW [g/mol] | C_{Stock} [mg/ml] | Lösungsmittel | Molverhältnis [mol/mol] | m [µg] | V [µl] |
|---|---|---|---|---|---|---|
| DOPE (Molekülsorte B) | 744,03 | 1 | Chloroform/EtOH | 1 | 515 | 515 |
| DOTAP (A) | 698,54 | 1 | Chloroform/EtOH | 1 | 485 | 485 |
| BFL-DHPE¹ | 1067,23 | 1 | Chloroform | 0,005 | 4 | 4 |
| Resveratrol (Polyphenol C) | 228,24 | 1 | EtOH | a. 0 | 0 | 0 |
| | | | | b. 0,62 | 100 | 100 |
| | | | | c. 1,25 | 200 | 200 |
| | | | | d. 2,5 | 300 | 300 |

| | | | | | | |
|---|---|---|---|---|---|---|
| ¹ nicht zur Molekülmischung zugehörig, sondern lediglich zum Nachweis der Fusion vorhanden. | | | | | | |

Es ergeben sich somit vier Ansätze mit einem Gesamtlipid aus Molekülsorte A, B und BODIPY® FL-DHPE/Resveratrol-Verhältnis von:
a) 2,005/0 mol/mol (Kontrolle)
b) 2,005/0,62 mol/mol
c) 2,005/1,25 mol/mol und
d) 2,005/2,5 mol/mol.

Die Komponenten wurden erst in Chloroform/Ethanol homogen vermischt. Nach der Mischung der Lipide mit dem Resveratrol wurden die organischen Lösungsmittel im Vakuum 30-60 min eingetrocknet und danach erneut in einer Pufferlösung von 20 mM HEPES (pH 7,4) in einer Endkonzentration von etwa 2 mg Lipid/ml Puffer aufgenommen und im Ultraschallbad 20 min homogenisiert. Die Emulsion liegt dann als Liposom haltbar mindestens 4 Wochen vor.

### Behandlung von 3T3-Zellen mit Resveratrolhaltigen Liposomen:

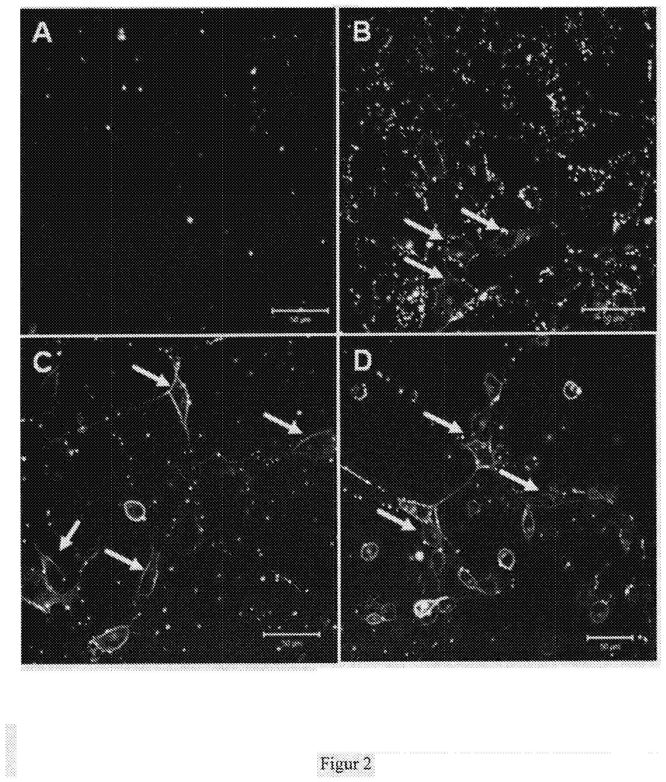

10 µl der Resveratrolhaltigen Liposomen wurden 100-fach mit RPMI Medium (Sigma-Aldrich, St. Louis, MO, USA) verdünnt und vor der Zugabe zu einer Zellkulturschale mit 3T3-Zellen (20.000-30.000 per Schale, (Durchmesser = 3,5 cm)) noch mal 5-10 Minuten lang mit Ultraschall (80-100W) behandelt. Anschließend wurden die Zellen mit HEPES-Medium gewaschen und die Fusionseffizienz des Reagenzes mit der Zellmembran am Fluoreszenzmikroskop überprüft. Figuren 2A-D sind die entsprechenden Ergebnisse zu den Experimenten a)-d). Der Balken gibt jeweils 50 µm an.

Die Experimente und die zugehörigen Figuren zeigen, dass eine sehr effektive und homogene Membranfusion ab einem Molverhältnis von Gesamtlipid/Resveratrol von 2,005/1,25 induziert wird (Figur 2 C), was durch den grün- fluoreszerienden Membranmarker BODIPY® FL-DHPE angezeigt wird. Dieser gibt in den in schwarz-weiss gehaltenen Figuren 2A-2D die Umrisse der Zellen durch Färbung der Membran, einige markiert durch die weißen Pfeile, an. Bei einer doppelten Resveratrol-Konzentration (Figur 2 D) ist die Zellmorphologie und die Anzahl der markierten Zellen unverändert.

Figur 2A zeigt, dass die positiv geladenen Liposomen ohne Resveratrol bzw. ein Polyphenol (DOPE/DOTAP/BODIPY FL-DHPE 1/1/0,005 mol/mol) keine Membranfusion mit 3T3-Zellen auslösen.

Figur 2B zeigt, dass ab einer Resveratrol-Konzentration von Gesamtlipid/Resveratrol=2,005/0,62 die erste inhomogene Fusion beobachtet werden kann. Ab dieser Konzentration ist in vitro und mit dem gewählten Nachweisverfahren eine Wirkung des Polyphenols C als Therapeutikum nachweisbar.

Figur 2C zeigt, dass eine Erhöhung der Resveratrol-Konzentration auf Gesamtlipid/Resveratrol=2,005/1,25 mol/mol die Fusionseffizienz der Liposomen deutlich erhöht. Ab dieser Konzentration des Resveratrol liegt man unter den gegebenen Untersuchungsbedingungen, das heißt in vitro, im optimalen Bereich einer Fusion.

Figur 2d zeigt, dass sogar beim doppeltem der Resveratrolkonzentration und einem Gesamtlipid/ Resveratrol-Verhältnis=2,005/2,5 neben einer hohen Fusionsrate eine unveränderte Zellmorphologie beobachtet wurde.

Darüber hinausgehende Konzentrationen wurden nicht mehr untersucht, da das Polyphenol C bei einem molekularen Verhältnis größer als 1:1:5 mol/mol nicht weiter effektiv in die Liposomen eingebaut werden kann und dadurch keine verbesserte therapeutische Wirkung im Vergleich zu neutralen Vesikeln nachgewiesen werden kann.

Es versteht sich somit, dass
1. Resveratrol bzw. das Polyphenol C in der Liposommembran selbst fusionsauslösend ist und
2. die in der wässrigen, liposomalen Phase vorliegenden Phenolanteile nach der Fusion in die Zelle und insbesondere in die Zellmembran überführt werden und dort effektiv als Therapeutikum für die auf oxidativem Stress beruhenden Erkrankungen, z. B. als Tumorbehandelndes Medikament, eingesetzt werden können und
3. in vivo mit deutlich verschobenen Konzentrationen des Polyphenols C gerechnet werden muss. Hier sollen molekulare Verhältnisse von A:B:C von 1(±0,2):1(±0,2):0,1-25 mol/mol bzw. die oben angegebenen Zwischenwerte gewählt und kombiniert werden, um das optimale Wirkergebnis für die entsprechenden Versuche zu erzielen. Die vorliegende Erfindung beschränkt sich auf molekulare Verhältnisse von A:B:C von 1(∓0,2):1(∓0,2):0,5-5 mol/mol.

Diese Maßnahmen stellen somit einen wirksamen Mechanismus bei der Behandlung aller oxidativer Erkrankungsprozesse in den Zellen dar.

### 2-84 Ausführungsbeispiel:

Es werden die übrigen in dieser Patentanmeldung offenbarten Ausführungsbeispiele für die Molekülsorte A und die Molekülsorte B und das Polyphenol C, wie in den Patentansprüchen 2 bis 4 angezeigt, miteinander kombinatorisch vermischt, wobei insgesamt 84 Kombinationen möglich sind. Die Molverhältnisse und Lösungsmittel entsprechen denjenigen des Ausführungsbeispiels 1. Dann erhält man die Molkülmischungen 2-84, die wie die Mischung 1 des Ausführungsbeispiels 1 als Liposom zur Anwendung in der Tumorbehandlung oder einer anderen oxidativen Erkrankung herangezogen werden können.

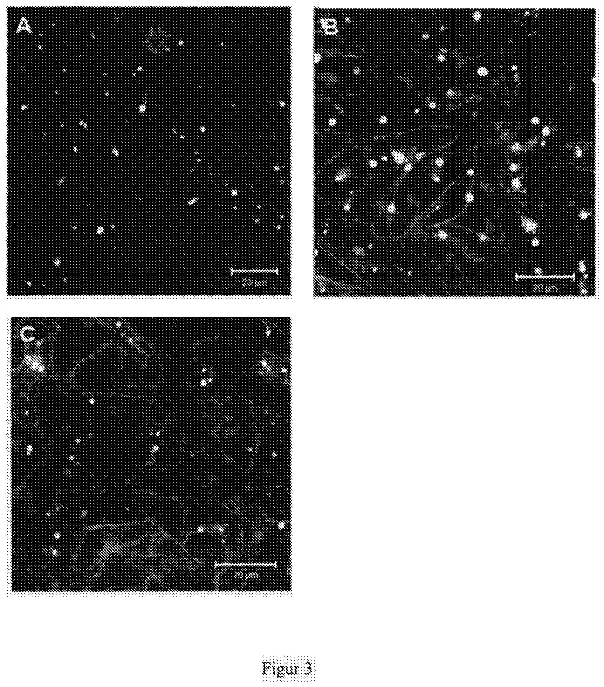

### Beispielhaft werden die folgenden weiteren Experimente genannt und deren Ergebnisse in der Figur 3 gezeigt:

Die in der Patentanmeldung neben Resveratrol aufgelisteten Polyphenole wie Curcumin und 5-Hydroxyflavon wurden auf CHO (Chinese Hamster Ovary) Zellen getestet, wobei Hydroxyflavon kein Polyphenol in Sinne der Erfindung ist. Die Liposomenpräparation und die Zellkulturverfahren wurden im Vergleich zu den vorangegangenen Experimenten nicht verändert:
Figur 3A zeigt, dass die positiv geladenen Liposomen ohne ein Polyphenol (DOPE/DOTAP/BODIPY FL-DHPE 1/1/0,005 mol/mol) auch keine Membranfusion mit CHO (Chinese Hamster Ovary) Zellen auslösen. Folgende Molekülmischung wurde hierzu verwendet: DOTAP:DOPE:BODIPY FL-DHPE von 1:1:0,005 mol/mol. BODIPY FL-DHPE ist nicht zur Molekülmischung zugehörig, sondern lediglich zum Nachweis der Fusion vorhanden.

Figur 3B zeigt, dass eine Erhöhung der Polyphenol-Konzentration, hier Curcumin, auf Gesamtlipid/Curcumin=2,005/1,25 mol/mol die Fusionseffizienz der Liposomen mit der Plasmamembrane der CHO Zellen ebenso deutlich erhöht wie Resveratrol. Folgende Molekülmischung wurde hierzu verwendet: DOTAP:DOPE:BODIPY-FL-DHPE:Curcumin von 1:1:0,005:1,5 mol/mol. BODIPY-FL-DHPE ist nicht zur Molekülmischung zugehörig, sondern lediglich zum Nachweis der Fusion vorhanden.

Figur 3C zeigt nach der Verwendung von 5-Hydroxyflavonhaltigen Liposomen (Gesamtlipid/5-Hydroxyflavon =2,005/1,25 mol/mol) an CHO (Chinese Hamster Ovary) Zellen eine ebenso hohe Fusionsrate wie mit den vorherigen Polyphenolen, wobei Hydroxyflavon kein Polyphenol in Sinne der Erfindung ist. Folgende Molekülmischung wurde hierzu verwendet: DOTAP:DOPE:BODIPY FL-DHPE:5-Hydroxyflavon von 1:1:0,005:1,5 mol/mol. BODIPY-FL-DHPE ist nicht zur Molekülmischung zugehörig, sondern lediglich zum Nachweis der Fusion vorhanden.

Die Aufgabe der Erfindung wird ferner durch Molekülmischungen und Verfahren zu deren Herstellung gelöst, bei denen die Molekülsorten A und B und das Polyphenol C in einem molekularen Verhältnis von 1(±0,2):1(±0,2):0,5-2,5 mol/mol, insbesondere in einem molekularen Verhältnis von A:B:C von 1(±0,2):1(±0,2):0,62-2,5 mol/mol und insbesondere in einem molekularen Verhältnis von A:B:C von 1(:1:0,2):1(:1:0,2):1,25-2,5 mol/mol vorliegen bzw. eingestellt werden.

Es ist denkbar, alle genannten Ausführungsbeispiele auch in pulverförmiger Tablettenform zu verabreichen, sofern auf die Überführung in die wässrige-liposomale Phase verzichtet wird.

## Patentansprüche

1. Molekülmischung für das Einbringen in lebende Zellen, umfassend
- eine amphipathische Molekülsorte A, welche im hydrophilen Bereich eine positive Gesamtladung aufweist und
- eine amphipathische Molekülsorte B, welche im hydrophilen Bereich neutral ist und
- ein Polyphenol C, welches mindestens zwei, direkt an einen aromatischen Ring gebundene Hydroxylgruppen enthält und zu den sekundären Pflanzenstoffen gehört,
- wobei die Molekülsorten A und B und das Polyphenol C in einem molekularen Verhältnis von A:B:C von 1(±0,2):1(±0,2):0,5-5 mol/mol vorliegen.

2. Mischung nach vorherigem Anspruch
**gekennzeichnet durch**
1,2-Dioleoyl-3-Trimethylammonium-Propan (DOTAP), N-(2,3-Dioleyloxypropyl)-N,N, N-Trimethylammonium Chlorid (DOTMA), Dimethyl-Dioctadecyl Ammonium Bromid (DDAB) oder (1-[2-(Oleoyloxy)Ethyl]-2-Oleyl-3-(2-Hydroxyethyl)Imidazolinium Chlorid (DOTIM) oder einer Mischung hieraus als Molekülsorte A.

3. Mischung nach einem der vorhergehenden Ansprüche,
**gekennzeichnet durch**
Phosphatidylethanolamine und Phosphatidylcholine, wie z. B. 1,2-Dioleoyl-sn-Glycero-3-Phosphoethanolamin, 1,2-Dipalmitoyl-sn-Glycero-3-Phosphoethanolamin, 1,2-Dimiristoyl-sn-Glycero-3-Phosphoethanolamin, 1,2-Dielaidoyl-sn-Glycero-3-Phosphoethanolamin, 1,2-Diphytanol-sn-Glycero-3-Phosphoethanolamin, 1,2-Dilinoleoyl-sn-Glycero-3-Phosphoethanolamin oder 1,2-Dioleoyl-sn-Glycero-3-Phosphatidylcholin oder einer Mischung hieraus als Molekülsorte B.

4. Mischung nach einem der vorhergehenden Ansprüche,
**gekennzeichnet durch**
Resveratrol, Curcumin und / oder Genistein oder einer Mischung hieraus als Polyphenol.

5. Mischung nach einem der vorhergehenden Ansprüche,
**dadurch gekennzeichnet, dass**
die Molekülsorten A und B und das Polyphenol C in einem molekularen Verhältnis von A:B:C von 1(±0,2):1(±0,2):0,5-2,5 mol/mol, insbesondere in einem molekularen Verhältnis von A:B:C von 1(±0,2):1(±0,2):0,62-2,5 mol/mol und insbesondere in einem molekularen Verhältnis von A:B:C von 1(±0,2):1(±0,2):1,25-2,5 mol/mol vorliegen.

6. Mischung nach einem der vorhergehenden Ansprüche,
**dadurch gekennzeichnet, dass**
diese als Lipsosom in wässriger Umgebung vorliegt.

7. Verfahren zur Herstellung einer Mischung nach einem der vorhergehenden Ansprüche 1 bis 5, **gekennzeichnet durch** die folgenden Schritte:
a) die Molekülsorten A und B werden in einem organischen Lösungsmittel gelöst,
b) das Polyphenol C wird von den Molekülsorten A und B getrennt in einem organischen Lösungsmittel gelöst und gemischt oder zusammen mit den Molekülsorten A und B in demselben organischen Lösungsmittel gelöst und gemischt,
c) sofern die Lösung des Polyphenols C in dem organischen Lösungsmittel in Schritt b) getrennt von der Lösung der Molekülsorten A und B erfolgte, werden das Lösungsmittel mit den Molekülsorten A und B und das Lösungsmittel mit dem Polyphenol C miteinander vermischt und homogenisiert, wobei in jedem Fall in den Schritten b) und c) ein molekulares Verhältnis von A:B:C von 1(±0,2):1(±0,2):0,5-5 mol/mol gewählt wird,
d) das bzw. die organischen Lösungsmittel werden entfernt.

8. Verfahren nach vorherigem Anspruch
**gekennzeichnet durch**
Wahl von Methanol oder Ethanol als Lösungsmittel für das Polyphenol C und durch Wahl von Chloroform, Ethanol und / oder Methanol als Lösungsmittel für die Molekülsorten A und B.

9. Verfahren nach einem der vorhergehenden zwei Ansprüche,
**dadurch gekennzeichnet, dass**
in den Schritten b) und c) ein molekulares Verhältnis von A:B:C von einem molekularen Verhältnis von 1(±0,2):1(±0,2):0,62-2,5 mol/mol und insbesondere in einem molekularen Verhältnis von A:B:C von 1(±0,2):1(±0,2):1,25-2,5 mol/mol gewählt wird.

10. Verfahren nach einem der vorhergehenden drei Ansprüche zur Herstellung des Liposoms nach Anspruch 6
**dadurch gekennzeichnet, dass**
die Molekülmischung in eine wässrige, physiologische Lösung überführt wird.

11. Medikament, umfassend eine Mischung nach einem der vorherigen Ansprüche 1 bis 6.

12. Mischung nach einem der vorherigen Ansprüche 1 bis 6
zur Verwendung als Medikament zur Behandlung von Zellerkrankungen, die durch oxidativen Stress verursacht werden.

13. Fusionsverfahren bei dem *in vitro* mit Polyphenol C beladene Liposome nach Anspruch 6 aufgrund der Wechselwirkung der positiv geladenen Lipide und der delokalisierten Elektronen der Polyphenole durch einfachen Oberflächenkontakt über wenige Minuten hocheffizient mit der Plasmamembran einer tierischen Zelle fusionieren.

## Claims

1. A molecule mixture for the insertion into living cells, comprising
- an amphipathic molecule type A which has a positive total charge in the hydrophilic region and
- an amphipathic molecule type B which is neutral in the hydrophilic region, and
- a polyphenol C which contains at least two hydroxyl groups directly attached to an aromatic ring and belongs to the secondary plant substances,
- wherein the molecule types A and B and the polyphenol C are present in a molecular ratio of A:B:C of 1(±0.2):1(±0.2):0.5-5 mol/mol.

2. A mixture according to preceding claim
**characterized by**
1,2-dioleoyl-3-trimethylammonium-propane (DOTAP), N-(2,3-dioleyloxypropyl)-N, N, N-trimethylammonium chloride (DOTMA), dimethyl-dioctadecyl ammonium bromide (DDAB) or (1-[2-(oleoyloxy)ethyl]-2-oleyl-3-(2-hydroxyethyl)Imidazolinium chloride (DOTIM) or a mixture thereof as molecule type A.

3. A mixture according to any one of the preceding claims,
**characterized by**
phosphatidylethanolamines and phosphatidylcholines such as 1,2-dioleoyl-sn-glycero-3-phosphoethanolamine, 1,2-dipalmitoyl-sn-glycero-3-phosphoethanolamine, 1,2-dimiristoyl-sn-glycero-3-phosphoethanolamine, 1,2-dielaidoyl-sn-glycero-3-phosphoethanolamine, 1,2-diphytanol-sn-glycero-3-phosphoethanolamine, 1,2-dilinoleoyl-sn-glycero-3-phosphoethanolamine or 1,2-dioleoyl-sn-glycero-3-phosphatidylcholine or a mixture thereof as molecule type B.

4. A mixture according to any one of the preceding claims,
**characterized by**
resveratrol, curcumin and/or genistein or a mixture thereof as polyphenol.

5. A mixture according to any one of the preceding claims,
**characterized in that**
the molecule types A and B and the polyphenol C are present in a molecular ratio of A:B:C of 1(±0.2):1(±0.2):0.5-2.5 mol/mol, in particular in a molecular ratio of A:B:C of 1(±0.2):1(±0.2):0.62-2.5 mol/mol and in particular in a molecular ratio of A:B:C of 1(±0.2):1(±0.2):1.25-2.5 mol/mol.

6. A mixture according to any one of the preceding claims,
**characterized in that**
it is present as a liposome in an aqueous environment

7. A method for the manufacture of a mixture according to any one of the preceding claims 1 to 5, **characterized by** the following steps:
a) the molecule types A and B are dissolved in an organic solvent,
b) the polyphenol C is separated from the molecule types A and B, dissolved and mixed in an organic solvent or dissolved and mixed together with the molecule types A and B in the same organic solvent,
c) if the solution of the polyphenol C in the organic solvent in step b) was carried out separately from the solution of the molecule types A and B, the solvent with the molecule types A and B and the solvent with the polyphenol C are mixed and homogenized with each other, wherein in each case, in the steps b) and c), a molecular ratio of A:B:C of 1(±0.2):1 (±0.2):0.5-5 mol/mol is selected,
d) the organic solvent or organic solvents are removed.

8. A method according to preceding claim, **characterized by** selection of methanol or ethanol as solvent for the polyphenol C and by selection of chloroform, ethanol and/or methanol as solvent for the molecule types A and B.

9. A method according to any one of the preceding two claims,
**characterized in that**
in the steps b) and c), a molecular ratio of A:B:C of a molecular ratio of 1(±0.2):1(±0.2):0.62-2.5 mol/mol and in particular in a molecular ratio of A:B:C of 1(±0.2):1(±0.2):1.25-2.5 mol/mol is selected.

10. A method according to any one of the preceding three claims for the manufacture of the liposome according to claim 6
**characterized in that**
the molecule mixture is converted into an aqueous, physiological solution.

11. A medicament, comprising a mixture according to any one of the preceding claims 1 to 6.

12. A mixture according to any one of the preceding claims 1 to 6 for use as a medicament for treating cell diseases caused by oxidative stress.

13. A fusion method in which, in vitro, liposomes loaded with polyphenol C according to claim 6 fuse highly efficiently with the plasma membrane of an animal cell due to the interaction of the positively charged lipids and the delocalized electrons of the polyphenols by simple surface contact over a few minutes.

## Revendications

1. Mélange de molécules à introduire dans des cellules vivantes, comprenant
- un type A de molécule amphipathique, qui a une charge d'ensemble positive dans la partie hydrophile et
- un type B de molécule amphipathique, qui est neutre dans la partie hydrophile et
- un polyphénol C, qui comporte au moins deux groupes hydroxyles liés directement à un cycle aromatique et qui appartient aux matières végétales secondaires,
- dans lequel les types A et B de molécule et le polyphénol C sont dans un rapport moléculaire de A:B:C de 1(±0,2):1(±0,2):0,5 à 5 mol/mol.

2. Mélange suivant la revendication précédente,
**caractérisé par**
du 1,2-dioléyl-3-triméthylammonium-propane (DOTAPE), du chlorure de N-(2,3-dioléyloxypropyl)-N,N-triméthylammonium (DOTMA), du bromure de diméthyl-dioctadécyl ammonium (DDAB) ou du chlorure de (1-[2-(oléoyloxy)éthyl]-2-oléyl-3-(2-hydroxyéthyl)imidazolinium (DOTIM) ou leurs mélanges comme type A de molécule.

3. Mélange suivant l'une des revendications précédentes,
**caractérisé par**
une phosphatidyléthanolamine et une phosphatidylcholine, comme par exemple la 1,2-dioléoyl-sn-glycéro-3-phosphoéthanolamine, la 1,2-dipalmitoyl-sn-glycéro-3-phosphoéthanolamine, la 1,2-dimiristoyl-sn-glycéro-3-phosphoéthanolamine, la 1,2-diélaidoyl-sn-glycéro-3-phosphoéthanolamine,la 1,2-diphytanal-sn-glycéro-3-phosphoéthanolamine, la 1,2-dilinoléoyl-sn-glycéro-3-phosphoéthanolamine ou la 1,2-dioléoyl-sn-glycéro-3-phosphatidylcholine ou leurs mélanges comme type B de molécule.

4. Mélange suivant l'une des revendications précédentes,
**caractérisé par**
du resvératrol, de la curcumine et/ou de la génistéine ou leurs mélanges comme polyphénol.

5. Mélange suivant l'une des revendications précédentes,
**caractérisé en ce que**
les types A et B de molécule et le polyphénol C sont dans un rapport moléculaire de A:B:C de 1(±0,2):1(±0,2):0,5 à 2,5 mol/mol, notamment dans un rapport moléculaire de A:B:C de 1(±0,2):1(±0,2):0,62 à 2,5 mol/mol et notamment dans un rapport moléculaire de A:B:C de 1(±0,2):1(±0,2):1,25 à 2,5 mol/mol.

6. Mélange suivant l'une des revendications précédentes,
**caractérisé en ce que**
il est présent sous la forme de liposome dans un environnement aqueux.

7. Procédé de préparation d'un mélange suivant l'une des revendications 1 à 5 précédentes, **caractérisé par** les stades suivants :
a) on dissout les types A et B de molécule dans un solvant organique,
b) on dissout et mélange le polyphénol C séparément des types A et B de molécule dans un solvant organique ou on le dissout et on le mélange ensemble avec les types A et B de molécule dans le même solvant organique,
c) dans la mesure où on a effectué la dissolution du polyphénol C dans le solvant organique au stade b) séparément de la dissolution des types A et B de molécule, on mélange entre eux et on homogénéise le solvant avec les types A et B de molécule et le solvant avec le polyphénol C, en choisissant en tout cas dans les stades b) et c) un rapport moléculaire de A:B:C de 1(±0,2):1(±0,2):0,5 à 5 mol/mol,
d) on élimine le ou les solvants organiques.

8. Procédé suivant la revendication précédente,
**caractérisé par**
le choix du méthanol ou de l'éthanol comme solvant du polyphénol C et par le choix du chloroforme, de l'éthanol et/ou du méthanol comme solvant des types A et B de molécule.

9. Procédé suivant l'une des revendications précédentes,
**caractérisé en ce que**
dans les stades b) et c), on choisit un rapport moléculaire de A:B:C de 1(±0,2):1(±0,2):0,62 à 2,5 mol/mol et notamment un rapport moléculaire de A:B:C de 1(±0,2):1(±0,2):1,25 à 2,5 mol/mol.

10. Procédé suivant l'une des trois revendications précédentes de préparation du liposome suivant la revendication 6,
**caractérisé en ce que**
on transfère le mélange de molécule dans une solution aqueuse physiologique.

11. Médicament comprenant un mélange suivant l'une des revendications 1 à 6 précédentes.

12. Mélange suivant l'une des revendications 1 à 6 précédentes, à utiliser comme médicament pour le traitement de maladies cellulaires provoquées par un stress oxydant.

13. Procédé de fusion *in vitro,* dans lequel, par des liposomes chargés de polyphénol C suivant la revendication 6, en raison de l'interaction des lipides chargées positivement et des électrons délocalisés des polyphénols, on fusionne par simple contact superficiel en peu de minutes, d'une manière très efficace, une cellule animale à la membrane plasmatique.
